# EUROPEAN PATENT APPLICATION

(11) **EP 4 700 388 A1**
(43) Date of publication of application: **25.02.2026**
(21) Application number: 24197935.0
(22) Date of filing: 02.09.2024
(51) Int. Cl.: G01N 33/52, B01L 3/00, G01N 21/78, G01N 33/72

(54) **DRY CHEMICAL DEVICE AND METHOD FOR TESTING ANALYTE IN URINE SAMPLE OR PROPERTY OF URINE SAMPLE**

(30) Priority: 20.08.2024 CN 202411152111
(71) Applicant: Zhejiang Orient Gene Biotech Co., Ltd, Anji Huzhou Zhejiang 313300 (CN)
(72) Inventor: FENG, Haiying, Huzhou, 313300 (CN); FANG, Jianqiu, Huzhou, 313300 (CN)
(74) Representative: Piotrowicz, Pawel Jan Andrzej

(57) **Abstract**

A testing device is disclosed in the present invention and includes a test strip, where the test strip includes a non-absorbent support sheet on which at least two or more testing areas are arranged, and liquid communication cannot be made between the testing areas, and the testing areas include an absorbent material thereon, and the absorbent material includes a reaction reagent that can detect an analyte in a urine sample or test the property of the urine sample; and the absorbent material, where the absorbent material is located under the support sheet; and when a liquid sample is dripped to each of the two or more testing areas, the absorbent material absorbs the excess liquid sample, thus avoiding interference of other testing areas where the liquid sample is not dripped.

## Description

### CROSS-REFERENCE TO RELATED APPLICATIONS

This application claims priority to the Chinese Patent Application, Application No. CN2024111521114, filed on August 20, 2024, and all disclosures of the Applications, including but not limited to the specification, abstract, claims and accompanying drawings of this application are hereby made a part of this application

### BACKGROUND OF THE INVENTION

### Field of the Invention

The invention relates to the field of in vitro detection, and in particular, to a device for testing an analyte in a urine sample and the property of the urine sample by a dry chemical method.

### Description of the Related Art

The following description is merely an introduction of some background knowledge and does not constitute any limitation to the invention.

The composition and physical characteristics of liquid are often used in test to understand the situation of the source of the liquid. For example, oxidants for testing biological liquids, pH value, specific gravity, creatinine, bilirubin, glucose, and other similar indicators can show health status or contamination. Food and beverage test items include pH value, specific gravity, contamination by bacteria or toxic substances, and the like. Test indicators of soil and water samples also include pH value, specific gravity, bacteria, lead or mercury contamination.

Generally, these rapid chemical tests can be accomplished by using test strips with a series of chemical test pads directly dipped with samples and reading testing results directly, without using capillary action. The test pad chemically reacts with the test sample or sample components to cause a color change. Usually, an operator only needs to immerse the test strips in the liquid, then observe the color change of the test pad, and compare the color of the test pad with that of the result card to determine the testing result.

However, such test strips of "dipping samples - reading results" have many disadvantages, and especially they cannot be combined with modern new rapid immunoassay devices. Nevertheless, these chemical tests are still necessary. The new rapid immunoassay devices are designed to reduce the contact opportunity between the operator and the samples under test. By using the testing method of "dipping samples - reading results", the operator must open the testing device, which will cause the samples to be contaminated by the operator or other reagents on the test strip.

These test strips of "dipping samples-reading results" are generally operated by professionals. Generally, the test strips with multiple test pads including ten or thirteen test pads are inserted into the urine sample and then taken out; and each test pad is compared with a standard color card in terms of color; then the testing results, such as positive or negative, are judged according to the color change obtained through such comparison. Sometimes, the testing results are directly inserted into a machine for automatic reading.

However, if the professional test is changed to home self-test or OTC products for being operated by the non-professionals, the biggest challenge is that due to the presence of multiple test pads, the non-professionals take more time to compare the color of each testing area with that of the standard color card, and these colors need to be read in a short time (within 2 minutes to 30 seconds; the testing results will be invalid if reading exceeds the time). For example, if there are 10 test strips, it is almost impossible for the non-professionals to read their results within one minute. Therefore, it is necessary to improve the design of such test strips and the testing methods, so as to meet the home self-test of non-professionals. On the one hand, such improvements need to meet the accurate operation and ensure the reliable and accurate testing results. On the other hand, such improvements need to ensure convenient operation and reading of the results without causing pollution and interference between testing areas not used.

Therefore, improved methods and devices to test multiple indicators of urine samples are very necessary.

### BRIEF SUMMARY OF THE INVENTION

For the disadvantages of conventional technologies, the invention provides a dry chemical test strip, and the test strip includes a plurality of testing areas that are sequentially arranged on a non-absorbent support sheet. These testing areas are arranged at intervals.

In some embodiments, the spacing between the testing areas is achieved by the non-absorbent support sheet. For example, the testing areas are arranged on the surface of the non-absorbent support sheet at intervals. In some embodiments, each testing area is provided with an absorbent pad, and the absorbent pad includes a chemical reagent and is used to test different analytes in a urine sample or the property of the urine sample.

For the convenience of the home test, in the invention, an operator is allowed to suck the urine sample through a pipette, drip it into one testing area, and then read the testing results for the testing area. After the testing results are read, the urine sample is dripped into the next testing area, so as to read the testing results for the next testing area. That is to say, the urine sample is dripped into one or two testing areas separately, and then the testing results for the one or two testing areas are read. Generally, the reading method is to compare the colors of the two testing areas with that of a standard color card to judge whether the testing results are negative or positive. After the testing results are read, the urine sample is dripped into another one or two test pads, and then the testing results for the another one or two test pads are read, so that home self-test can be realized. This operation mode is an "intermittent" operation mode rather than a mode where the urine sample is dripped into all test pads at one time. Then, the testing results are compared in sequence. To be specific, in the operation mode, the urine sample is dipped into some test pads. After the above test pads are tested and the testing results thereof are read, the urine sample is dripped into another test pads on a same test strip and the testing results are read. This testing way is an intermittent testing way.

In some embodiments, an absorbent material is provided under the test strip. If too much urine is dripped, the excess urine will flow to the absorbent material and be absorbed, so as not to pollute other test pads that are not dripped. In some embodiments, the test pad is dry when there is no liquid and before the test, and it will become wet after contacting the liquid and cannot absorb the liquid.

In some embodiments, for the test strip of the invention, a non-absorbent support sheet is provided with a plurality of testing areas that are respectively provided with one absorbent test pad, and each test pad is used to treat different kinds of chemical substances, such that the contents of different analytes in urine samples or the properties (PH value and gravity) of the urine samples are tested through chemical reaction. The distance between each test pad is about 3-5 mm, and the total length of the test strip is about 15-20 cm. If there is a hand-held part, the length of all testing areas is about 8-10 cm. In this case, if the test strip is directly exposed, the operator is allowed to drip the urine sample into each test pad in sequence, and the testing results are read after 1-2 testing pads are dripped. Generally, the test strip is compared with the standard color card in terms of color, and then the testing results are recorded. Although this method is feasible, it is still not very convenient to operate. In an operator occasion, there are containers containing urine, droppers, absorbent paper spread on a table, and standard color cards, all of which are placed in a same place. Moreover, the test pads are concentrated on a slender strip and relatively close to each other. In a dripping process, the urine sample may be dripped in a wrong order, for example, the urine sample originally dripped into the first test pad may be dripped into the second test pad or the third test pad; alternatively, the urine sample originally dripped into the second test pad or the third test pad may be dripped into the fourth test pad or the fifth test pad due to the wrong operation; such operation is easy to cause confusion and wrong comparison when the testing results for the test pad are compared with these on the standard color card. Although self-test can be realized by using a single test strip and changing a liquid application way, there are still some inconveniences in operation.

In some embodiments, in order to limit the dripped urine sample to only one or two testing areas, the testing areas are paced apart each other by using some ways to avoid dripping the urine sample on the testing areas that are undesired to be tested immediately. In addition, the dripped urine sample is only dripped into the testing areas required for immediate testing, and those areas required for subsequent testing do not receive the urine sample. That is to say, for example, there are 10, 13 and 15 testing areas spaced apart on the test strip. Therefore, the way of dripping the urine sample by a non-professional person at home under direct guidance instead of randomness is needed. The reason is that the operator always starts dripping from one end and ends up such dripping at the other end, and such operation is performed in a certain single order, which can avoid mistakes and cross-contamination. Therefore, in one embodiment, the testing areas are spaced apart each other, for example, in some specific embodiments, the testing areas are separated by spacing grids, such that the testing areas are independent, and the adjacent testing areas will not contact liquid in advance when the urine sample is dripped into the testing areas. There are many ways to make the testing area or a plurality of test pads remain relatively independent during sample dripping and testing. These ways can achieve independent reception of liquids, independent test, and independent reading of testing results within the effective time, and these operations do not interfere with each other.

There are many ways to make the test pads independent and avoid mutual interference. For example, in some embodiments, the testing area of the test strip is covered with a film, for example, the test pad is covered with a film, and the film can protect the test pad and make the operation more convenient during testing. In some embodiments, the test pad on each test strip is covered with a waterproof film. If the film is flexible, before one test pad is dripped every time, the film is uncovered to expose the test pad, the urine sample is dripped, and then the testing results are read. When it is necessary to continue the next test, the film covering the next test pad is uncovered. This way can be called a "sequential and interval" way. In some embodiments, each test pad can be covered with a single film, or one film can be used to cover multiple test pads, and then the film can be covered on the surfaces of the test pads by hot pressing, such that the film can be adhered to the spacing areas between the test pads. The film is sequentially uncovered from one end, the test pads are sequentially exposed, and the urine sample is sequentially dripped on the exposed test pads, thus completing the test or assay on the test pads. In this way, the "interval" time between the test pads can be controlled; and after the previous test pad is tested and the testing results thereof are read, the next test pad is tested and the testing results thereof are read. Thus, the dripping of the test pad can be controlled by different people according to their familiarity with the operation, just by following the instructions provided. For example, when leukocytes are tested, the testing results are read within 120 seconds after dripping, and then the liquid sample is dripped into the next test pad for testing.

In some embodiments, another alternative way to cover the film is to provide a transparent sliding cover; before the test, the cover completely covers the test pad; and when testing is needed, the cover can slide. In a sliding process, the test pads are exposed and tested sequentially and at intervals. In some embodiments, the sliding cover has a bottom board, and the test strip is arranged on the bottom board. In some embodiments, a layer of absorbent paper is arranged on the bottom board, the test strip is arranged on the absorbent paper, and the bottom board is provided with a chute, and the sliding cover moves in the chute to sequentially expose the test pad.

In other embodiments, the test strip is arranged in one device, the device is provided with windows for dripping liquid, and one or two test pads are exposed of each window. In addition, there is a spacing structure between the windows; and the spacing structure is arranged between the test pads such that the test pads are spaced apart each other. The spacing structure is arranged between the test pads and on the non-absorbent support sheet on the test strip. In some embodiments, the spacing structure includes a baffle that is arranged between the test pads, such that a plurality of test pads can be divided into a plurality of independent units; for example, each test pad can be an independent unit, and two test pads can also be divided into one unit. Thus, the test pad is tested from one end to the other end sequentially and at intervals, for example, the urine sample is dripped onto a first test pad in a first window, and then the testing results are judged by comparing the color of the test pad with that of the standard color card. After the test, the urine sample is dripped onto the test pad in a second window adjacent to the first window, and then the testing results are judged by comparing the color of the test pad with that of the standard color card. It takes a certain time to compare the color of the test pad with that of the standard color card to obtain the testing results; generally, the time is within 120-30 seconds. For example, in some tests, the reading results are valid when read within 120 seconds; and in some tests, the testing results are valid when read within 60 seconds, 45 seconds, 40 seconds and 30 seconds. In some embodiments, the time to read the testing results of the test pads is arranged from long to short in this sequential and interval way, for example, the time to effectively read the testing results of the test pad where the urine sample is firstly dripped is longer than the time to effectively read the testing results of the test pad where the urine sample is finally dripped. Thus, a test subject can have enough time to read the testing results. In addition, for some primary operators, the long initial test time and the short later test time more conform to their operation habits, such that the operators can gradually become familiar with the operation process and the sequential and interval way.

In some embodiments, the window is arranged in an upper board, and the spacing structure is also arranged on the upper board and extends from the upper board, and a lower board is also provided, and the lower board is used for placing the test strip. When the upper board and the lower board are combined by sockets and bolts, each test pad or every two test pads are separated by the window, and every two spacing elements are arranged between the test pads, such that a plurality of test pads are divided into a plurality of independent testing units through the spacing structure and the window, and one or two test pads are arranged in each testing unit (each window). When testing is needed, the urine sample is directly dripped into the window and flows to the test pad, the color of the test pad is compared with that of the standard color card and the testing results are read. In some embodiments, the standard color card is printed on the surface of the window near the test pad, and corresponds to the color block of the test item. Generally, when the color of the test pad changes, the testing results can be judged to be positive or negative by directly comparing the color of the test pad with the color of the color block on the surface of the window. This convenience lies in that the window is the entrance for dripping the urine sample and the reading area. In addition, the test pad in a single window receives the urine sample, without affecting the test pads in other windows; and the urine sample will not flow to other test pads. Thus, the operator can operate in a sequential and interval way. For example, after the test pad in one window is tested and read, the test pad in the next window is tested. In this way, the so-called next window is not adjacent, and can be selected at will. After all, each window exists independently, which makes it more convenient for operators and increases randomness.

In some embodiments, a film is covered on the upper board (specifically on the window), so as to protect the test pad under the window, avoid moisture regain and play a protective role. For example, when liquid is dripped into the first window, the film is uncovered, but the adjacent window is covered with the film and the liquid will not be dripped into the adjacent window. Although the test pads are separated by the window, the distance between them is still relatively close. Covering with an impermeable film can also prevent the liquid from being dripped at a wrong position, and also avoid the mutual pollution of the liquid between the test windows. The film can cover multiple windows as a whole, or a window can be covered with a film. When the test pad in one window is tested every time, the film of the window is uncovered or sequentially uncovered once.

In some embodiments, in order to keep the liquid dripped onto the test pad constant in volume and not excessive, a layer of filter paper is arranged under the support sheet, absorbs excess liquid, and is not in contact with the test pad. In some embodiments, the test pads are vertically divided into independent units by the partition structure of the upper board. When two or more test pads are located in a window and when the first test pad is dripped, the excess liquid will flow to another test pad in the same window, so the width of the window is larger than that of the non-absorbent support sheet, and there is a gap between the window and the support sheet. If there is excess liquid, the liquid will flow to the absorbent paper through the gap, thus reducing the excess liquid flowing to the test pad in the same window. Therefore, in some preferred embodiments, only one test pad is arranged in each window, such that the testing results of the test pad of the window are completed independently by dripping the liquid to the window, and then the liquid is dripped to other windows. Multiple test pads are tested in an "intermittent" way.

Another aspect of the invention provides a method for analyzing a liquid sample. The method includes: providing the device; dripping a liquid sample into a test pad sequentially and at intervals; allowing the test pad to react with the liquid sample for a long enough time to generate a detectable signal; and comparing the signal with a standard signal. In some specific embodiments, the detectable signal may be a change in the color of the test pad. The "standard" may be any appropriate and objective way of expressing a testing result. For example, the standard may be a standard comparison table or card provided for the device, or together with the device, or in other possible ways, and the comparison of the signal with that described in the standard includes the comparison of the color of the test pad after the test with the color of the standard table or card, and determining the testing results through such comparison. The detectable signal may be indicative of any objective analytical result (for example, fluorescence, enzyme-based assay, or spectrophotometric assay). The standard can be made based on any of these or other testing methods. These methods include: reacting long enough to allow the liquid sample to react with the reagents on the test pad, and comparing the color of the test pad with the color in a standard result comparison table or card at the end of the reaction.

In some embodiments, the so-called sequential and interval way is that the liquid sample is sequentially dripped into the test pads according to the arrangement order of the test pads, for example, sequentially dripping the liquid sample into the test pads and then waiting for the testing results. The interval here indicates that the dripped test pads are some of multiple test pads, for example, one or two of 13 or 12 test pads, at least less than 13 or 10 test pads. In addition, it is necessary to drip the remaining test pads or the remaining parts of the test pads after the dripped test pads are tested. For example, there are 10 test pads on the test strip, and the liquid can be dripped into one test pad, and then dripped into another test pad after the test pad reacts with the liquid and the testing results are read, and then dripped into the third or fourth test pad after the another test pad is tested. Here, the "end of test" or "completion of test" means that the reaction and reading of the test pad are completed after the test pad receives the liquid.

The invention also provides a kit for testing a liquid sample. The kit includes the device of the invention and an operation instruction of the device. In different specific embodiments, the kit can also include an analysis result comparison table or other standards. The kit can be packaged in any suitable form, such as vacuum sealed boxes, plastic bags or aluminum foil bags.

The invention further includes some other useful aspects described in detail here. These aspects can be fully understood by using these products. These aspects need to be further combined with various specific embodiments for investigation to obtain complete evaluation in the invention. In addition, other aspects and specific embodiments of the invention will also be described in detail.

The summary of the invention is not limited to the above description, and other features and benefits of the invention are reflected in the following detailed description and claims.

### BRIEF DESCRIPTION OF THE DRAWINGS

FIG. 1 is a structural schematic diagram (including 13 test indicators) according to a specific embodiment of the invention.
FIG. 2 is a structural schematic diagram (including 10 test indicators) according to a specific embodiment of the invention.
FIG. 3A is a schematic diagram showing an operation process according to a specific embodiment of the present invention.
FIG. 3B is a schematic diagram showing a comparing process of a test strip and a color card according to a specific embodiment of the invention.
FIG. 4 is a top view showing a testing device according to a specific embodiment of the invention.
FIG. 5 is a structural schematic diagram of a testing device according to a specific embodiment of the invention.
FIG. 6 is a schematic diagram showing an exploded structure of a testing device according to a specific embodiment of the invention.
FIG. 7 is a schematic diagram showing a lateral cross-section structure of a testing device according to a specific embodiment of the invention.
FIG. 8 is a schematic diagram showing a longitudinal section of a testing device according to a specific embodiment of the invention.
FIG. 9 is a schematic diagram showing an exploded structure of a testing device according to a specific embodiment of the invention.
FIG. 10 is a structural schematic diagram of a testing device according to a specific embodiment of the invention.
FIG. 11 is a structural schematic diagram showing a section of a testing device according to a specific embodiment of the invention.
FIG. 12 is an exploded view showing a three-dimensional structure of a testing device according to a specific embodiment of the invention.
FIG. 13 is a schematic diagram showing a three-dimensional structure of a testing device according to a specific embodiment of the invention.
FIG. 14 is a schematic diagram showing an assembling structure of a testing device according to a specific embodiment of the invention.
FIG. 15 is a schematic diagram showing an assembling structure of a testing device according to a specific embodiment of the invention.
FIG. 16 is a structural schematic diagram showing a lateral section according to another embodiment of the invention.
FIG. 17 is an exploded view showing a three-dimensional structure of a testing device according to a specific embodiment of the invention.

### DETAILED DESCRIPTION OF THE INVENTION

In the following detailed description, the accompanying drawings and corresponding textual descriptions are only intended to illustrate particular specific embodiments that are likely to be implemented in the invention. We do not exclude that the invention may also be implemented in other specific embodiments and that the structure of the invention may be altered without violating the application scope of the invention.

### Detection

Detection means to assay or detect presence or absence of a substance or material, including but not limited to, a chemical substance, an organic compound, an inorganic compound, a metabolite, a drug, a drug metabolite, an organic tissue, a metabolite of an organic tissue, a nucleic acid, a protein, or a polymer. In addition, detection means that the amount of a substance or material is tested. Further, assay also means immunoassay, chemical assay, enzyme assay, and the like. In a specific embodiment of the invention, a reaction pad or a test pad is used to test the property of a sample or the presence or absence of an analyte through chemical reaction, and the analyte can also be tested through an immune method.

### Samples

Samples tested by the testing device of the invention include biological fluid (for example, case fluid or clinical sample). Liquid samples or fluid samples may be derived from solid or semi-solid samples, including feces, biological tissues and food samples. The solid or semi-solid specimens may be converted to liquid specimens by any appropriate methods, such as mixing, mashing, macerating, incubating, dissolving, or digesting the solid specimens by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological specimen is urine; and preferably, the biological specimen is saliva. Food samples include food processing substances, final products, meat, cheese, wine, milk, and drinking water. Plant specimens include specimens derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other wastewater.

In some embodiments, the fluid sample needs to be manually dripped by the testing element of the invention, such as dripping the liquid sample to the test pad with a dropper to test the analyte in the liquid sample or the property of the liquid sample. In some embodiments, the sample of the present invention may be a urine sample.

### Downstream and upstream

Downstream or upstream is divided according to a flow direction of a liquid, generally, a liquid or fluid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to facilitate the flow of a liquid, a liquid may overcome gravity to flow towards an opposite direction to the gravity; and in this case, downstream or upstream is divided according to a flow direction of the liquid. For example, in the testing device of the invention, when there is the liquid sample at the bottom of the testing chamber, for example, the liquid sample from the sample chamber, such as a urine sample or a saliva sample of a test subject, can flow from an end of the test strip to the other end of the test strip (that is to say, from the bottom to the top).

### Liquid communication

Liquid communication means that liquid or gas can flow from one place to another place. In a flow process, the liquid or gas may pass through some physical structures that play a guiding role. The "pass through some physical structures" here means that the liquid passes through the surfaces of these physical structures or their internal space and flows to another place passively or actively, where such passive flow is usually caused by external forces, such as flow under the capillary action and the action of air pressure. The flow here may also be an active flow of the liquid due to self-action (gravity or pressure), and also may be a passive flow therefore. The fluid may be a forward flow or also a reverse flow under the action of air pressure; or the fluid is caused to flow from a position to another position under the action of air pressure. The communication here does not mean that a liquid or a gas is necessarily present, but indicates a relationship or state between two objects under some circumstances. If a liquid is present, it can flow from one object to another. Here it means the state in which two objects are connected. In contrast, if there is no liquid or gas communication state between two objects, and a liquid exists in or on one object but is unable to flow into or on another object, it is a non-communication, non-liquid communication or non-gas communication state.

### Analyte

Examples that can use an analyte related to the invention include some small-molecule substances, including drugs (such as drug of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of drug abuse include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzodiazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The testing device of the invention may also be used to detect drugs belonging to a medical use but easy to be taken excessively, such as tricyclic antidepressants (imipramine or analogues) and acetaminophen. These drugs are metabolized into micromolecular substances after being absorbed by human body. These micromolecular substances exist in blood, urine, saliva, sweat and other body fluids or in some body fluids. For example, the analyte detected by the invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. Any other clinical chemistry analysis may be performed by lateral flow test in combination with the device of the invention. The sample of the invention may be urine, and the analyte may be HCG, LH, and other substances, which are used for testing ovulation or early pregnancy.

In another example, the test strip or testing device of the invention can be used to test the following liquid sample, for example, the nature of the analyte in the urine or liquid. In an example, the following analyte and property components can be tested.

Glucose (GLU) was tested with an enzymic method, and two enzymes were used, namely glucose oxidase and catalase. At present, almost all test strips are tested with an enzymic method, because the enzymic method features such advantages as strong specificity, high sensitivity and short reaction time. An indicator selected for a different size of test strip is different. However, there are two modes of making methods for the test pad for glucose test. It is required to keep enzyme activity as the enzymic method, is used, and there shall not be too many samples; otherwise, the color will become too dark to test. Therefore, a small amount of liquid is generally required. However, some hydrophobic substances need to be added to make an enzyme stay active. These hydrophobic polymers can keep the enzyme continuously active, for example, by adding carboxymethyl cellulose and other substances. However, when these hydrophobic substances are used to treat the test pad, a hydrophobic membrane is formed on the test pad. When liquid is added to the surface of the test pad, the liquid does not naturally spread out. Therefore, when the liquid is dripped into the test pad, the bottom of the dropper needs to be in contact with the test surface, to make the liquid spread out on the surface of the test pad. In addition, when this indicator is used for test, it is not desirable to load too many samples onto the test pad, for example, an acidic substance, nitrogen and other substances in some urine will affect a function of the enzyme. Therefore, this test indicator always comes last and is not the first indicator to be tested. In addition, the valid reading time of the indicator is the shortest, being basically within 30 seconds. In a process of testing the first indicator to the tenth or thirteenth indicator, the operator is already familiar with and accustomed to the test. In this way, the indicator with the shortest reading time being arranged the last one to be tested is also a preferred embodiment of the invention.

The significance of testing blood samples has the following aspects: first, physiological diabetes is transient diabetes, which is temporary and returns to normal after physiological factors have been excluded. There are three main types of physiological diabetes: (1) Alimentary diabetes, namely hypergloycemia caused by taking a lot of sugar in a short period of time; (2) Stress diabetes, namely transient diabetes occurred after abnormal secretion of adrenal hormones or insulin due to the stimulation of the sugar center of the medulla oblongata in the case of brain injury, cerebrovascular accident, emotional excitement, and periodic quadriplegia caused by strenuous exercise; (3) Diabetes usually seen during middle and late pregnancy. Second, pathological diabetes can also be classified into three types: (1) pancreatic diabetes, referring to the diabetes in which the secretion of insulin is relatively or absolutely insufficient, so that the blood glucose concentration exceeds the renal sugar threshold. This case can not only help to diagnose diabetes, but also guide clinicians to decide the dosage of insulin and judge the curative effect; (2) renal diabetes, referring to the diabetes in which the renal tubular reabsorption of glucose is decreased and which can occur even if the proximal convoluted tubule function of newborn is not perfect; and (3) other diabetes, referring to the diabetes in which the blood glucose concentration is higher than the renal glucose threshold due to excessive growth hormone (acromegaly), excessive thyroid hormone (hyperthyroidism), excessive adrenal hormone (pheochromocytoma), cortisol (cushing syndrome), glucagon and other factors; in addition, obesity and hypertension may also lead to diabetes. A subsequent reaction after urine glucose analysis is an oxidation-reduction reaction. Therefore, when the urine contains a substance with stronger pigment reducing ability, the testing result may be low or even false negative. For example, when the urine contains vitamin C, the testing result will be low or even false negative. Therefore, some of the above defects are avoided through liquid dripping. This indicator is set last out of consideration for interference from other substances. Therefore, excessive urine is not expected to be absorbed by the test pad, and more urine absorbed indicates a greater interference. Besides, the time of reading being kept within 30 seconds also aims to minimize the interference of these interfering substances. After all, it takes time for these interfering substances to react with enzymes and substrates. Therefore, a shorter reading time is beneficial to the accuracy of the testing result.

Bilirubin (BIL) and urobilinogen (URO). Under acidic condition, diazonium salt acts on the center of the bilirubin, disconnects the bilirubin and the disconnected bilirubin couples with the diazonium salt to form 2 molecules of azobilirubin, thus causing color change. Generally, a test strip for urobilinogen analysis has two reaction principles, one of which is that urogenin reacts with p-dimethylaminobenzaldehyde under an acidic condition (that is, urobilinogen condenses with aldehyde to produce a red acetal compound, which is a common Euclidean reagent); and another of which is a diazonium salt method, in other words, urobilinogen is coupled with diazonium salt under an acidic condition to produce a purple azoic compound. Clinical significance: 1. Bilirubin testing is of significance to diagnosis of diseases of the liver and biliary system, and urobilinogen reflects a liver function more sensitively. 2. Bilirubin testing helps to diagnose jaundice. In the case of septicemia, bean disease and abnormal blood transfusion, a large number of red blood cells are destroyed, resulting in hemolytic jaundice. At this time, although bilirubin has increased greatly, most of it is indirect bilirubin, so bilirubin in urine is still negative. 3. The test of a bilinogen group can sensitively reflect a hepatocyte function and clinical experience shows that the bilinogen group in urine has increased significantly before jaundice appeared in the early stage of viral hepatitis. A combination with bilirubin provides basis for diagnosis of a type of jaundice. Jaundice can be broadly divided into three categories: (1) Prehepatic jaundice, or hemolytic jaundice. (2) Hepatogenic jaundice, or hepatogenic jaundice, which is caused by lesions of a large number of liver cells or intrahepatic capillary bile ducts under such conditions as infection (such as viral hepatitis), intoxication and cirrhosis, which makes the liver cells hinder the uptake, binding, transport and excretion of bilirubin. (3) Posthepatic jaundice, or obstructive jaundice, which is caused by obstruction of common bile duct as a result of stones, tumors or congenital biliary atresia. Precautions: 1. The sample must be fresh to prevent bilirubin from being oxidized to biliverdin, and strong sunlight will accelerate this reaction. After being left for a long time, urobilinogen can be oxidized into urobilin. 2. High levels of vitamin C or nitrite in the urine inhibits the diazo coupling reaction and may result in a false negative result. A false negative result may be shown when a patient is treated with a large dose of chlorpromazine or urine of such patient contains a metabolite product of phenylazopyridine hydrochloride. 3. There is no negative gradient of urobilinogen on the analytical test strip, therefore, the test strip cannot be used to detect the decrease or disappearance of urobilinogen. 4. Some endogenous substances such as porphobilinogen, indole, and bilirubin in urine may lead to a false negative result; and drugs such as phenothiazine may cause color interference. In addition, high levels of vitamin C or nitrite in the urine inhibits the diazo coupling reaction and may result in a false negative result.

Urine ketone bodies (KET). Urine ketone bodies include acetoacetic acid, acetone and beta-hydroxybutyric acid, the latter two of which are not ketone, but are often accompanied by the first two, so all of which are collectively known as ketone bodies. The reaction principle is that acetoacetic acid and acetone in urine react with sodium nitrosferricyanide to generate a purple-red complex. This method has sensitivity to acetoacetic acid of 5~10 mg/dl and sensitivity to acetone of 40-70 mg/dl. Besides, sodium nitrosferricyanide does not react with beta-hydroxybutyric acid. Clinical significance: 1. Diabetic ketoacidosis. The utilization of glucose is reduced, and the decomposition of fat produces excessive ketone bodies. An examination of urine ketone body is of great value to diagnosis of acidosis or coma due to uncontrolled or improperly treated diabetes, which is distinguished from acidosis caused by cardiac and cerebral disease or coma caused by hyperglycemic osmotic diabetes. 2. Infectious diseases (such as pneumonia, typhoid fever, septicemia, tuberculosis and other fever periods), severe vomiting, diarrhea, chronic hunger, fasting, general anesthesia and other diseases may all lead to ketonuria. In addition, ketonuria may also occur to pregnant women who vomit more, eat less and have significantly increased body fat metabolism due to a pregnancy reaction. 3. Phosphorus poisoning caused by anesthesia with chloroform and ether can also give rise to ketonuria. 4. Ketonuria may also occur after taking biguanide hypoglycemic drugs such as phenformin, because the drugs inhibit cell respiration. Precautions: Acetone and acetoacetic acid in the urine ketone body are volatile substances; acetoacetic acid is easily decomposed into acetone by heat; and acetone disappears after the urine is contaminated with bacteria. Therefore, the urine must be fresh and tested in time, to avoid a low testing result or false negative result. Sensitivity of the dry chemical method for ketone body determination to acetoacetic acid is 7-10 times that to acetone. Therefore, the method is different from other test methods

Specific gravity (SG). Reaction principle: A reaction principle of a test strip for urine specific gravity is the ion exchange method, in which polymer electrolyte, a copolymer of methyl vinyl ether and maleic acid, is a weakly acidic (-COOH group) ion exchanger. But electrolyte (M+X-) in the form of salt in urine dissociates in urine and releases M+ cations (mainly Na+) which exchange with hydrogen ions in the ion exchanger and release H+ ions. The H+ ions make the pH indicator bromothymol blue change color (from green to yellow). Clinical significance: a renal concentration function can be estimated by the determination of urine specific gravity. As the urine specific gravity is also influenced by age, water intake and sweating, multiple determinations can better reflect the renal concentration function than a single determination. Precautions: 1. The urine specimen must be fresh and shall be free from a strong alkali substance, a strong acid and other substances (such as quinine and pyrimidine), because presence of these substances affects the determination of urine specific gravity. When urine pH is greater than 7, 0.005 should be added to the determination results as a correction for alkaline urine. 2. The urine analysis test strip actually determines ion concentration in urine, thus a non-ionic compound (e.g. glucose and a contrast agent) must have some influence on the determination result.

Occult blood (BLD). Reaction principle: a reaction principle of a test strip for urine occult blood analysis is that peroxides are catalyzed and decomposed by using pseudoperoxidase activity of heme in hemoglobin and new ecological oxygen is produced. The oxygen oxidizes the indicator and makes the indicator develop color, and concentration of blood in urine can be obtained from intensity of the color development. Clinical significance: 1. More than 0.1% blood mixed in urine is presented with a gross hematuria feature, and an amount of blood below this level can only be proved by an occult blood reaction or urine sediment microscopy. Hematuria is common in urinary tract inflammation (acute nephritis, renal tuberculosis, urethritis, and the like), tuberculosis and tumor; inflammation occurs in case of leukocytes; and nephritis may occur when protein is positive and there are renal epithelial cells and casts in urine sediments. Especially when there are red cell casts, renal parenchymal hemorrhage is demonstrated. 2. Hemoglobinuria is found in paroxysmal hemoglobinuria, various poisoning, infection, streptococcal septicemia, malaria (hemoglobinuric fever), burns, and hemolytic transfusion reactions. Precautions: 1. Menstrual blood of adult women can often cause false positive testing results, so necessary urine collection measures should be taken to reduce pollution. 2. Analytical test paper can test blood and hemoglobin. Therefore, broken hemoglobin may cause the testing results of the test paper to be inconsistent with those of microscopic examination, and the testing results here should be distinguished. 3. Urine including heat labile enzymes, myoglobin or bacteriuria can cause false positive; urine including a large amount of vitamin C can inhibit later reaction, such that the testing results are relatively low or even false negative. Therefore, analytical test paper with anti-interference ability of vitamin C should be used in the test.

Principle of PH detection: acid-base indicator method; reagent composition: methyl red and bromothymol blue. Reaction process: two acid-base indicators (methyl red and bromothymol blue) in the reagent show color reaction when contacting with urine samples. Color range: PH5-9 changes from acidic to alkaline in order of orange, green and blue.

Protein (PRO): detection principle: protein error method of acid-base indicator; reagent composition: bromophenol blue acid-base indicator, citric acid buffer system and surfactant. Reaction process: the bromophenol blue acid-base indicator in the reagent reacts with protein (mainly albumin) in urine samples upon contact, with the color change; and the color depth is directly proportional to the content of protein (mainly albumin).

Nitrite (NIT): detection principle: nitrite reduction method; reagent composition: 4-aminophenylarsonic acid, and 1,2,3,4-tetrahydrobenzo[h]quinoline. Reaction process: 4-aminophenylarsonic acid in the reagent reacts with nitrite in urine samples to form diazonium salt upon contact; and then the diazonium salt reacts with 1,2,3,4-tetrahydrobenzo[h]quinoline to generate red compounds, and the color depth is in direct proportion to the content of nitrite.

Other chemical testing methods of other analytes not described herein are all available in the prior art and are all conventional methods.

### Testing element

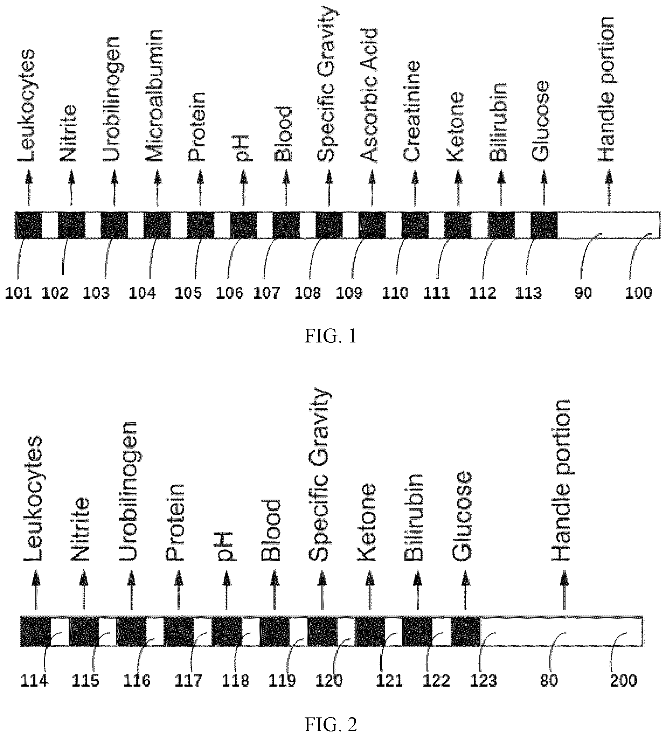

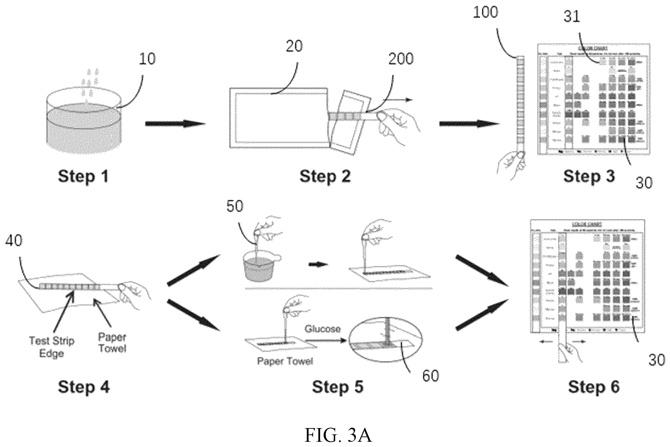

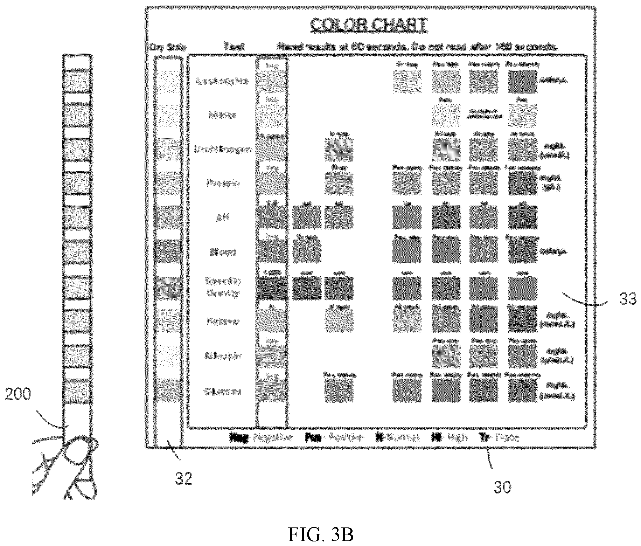

FIG. 1 shows a test strip 100 according to a specific embodiment of the invention. The test strip includes a non-absorbent support sheet 90 on which test pads are arranged sequentially and at intervals from one end to the other end. The test pads are made of absorbent materials and respectively correspond to one test indicator. For example, as shown in FIG. 1, there are 13 test pads, where each test pad corresponds to one test indicator, including from left to right: a test pad 101 for testing leukocytes, a test pad 102 for testing nitrite, a test pad 103 for testing urobilinogen, a test pad 104 for testing microalbumin, a test pad 105 for testing protein, a test pad 106 for testing PH value of sample, a test pad 107 for testing blood, a test pad 108 for testing specific gravity of urine, a test pad 109 for testing ascorbin acid, a test pad 110 for testing creatinine, a test pad 111 for testing ketone, a test pad 112 for testing bilirubin, and a test pad 113 for testing glucose. These test pads are distributed at a certain distance, and are separated by non-absorbent support sheets (parts of the support sheets 114, 115 as shown in FIG. 2 are partial areas of the non-absorbent support sheets 114, 115 to separate the test pads); then a blank area 90 reserved on a left side serves as a hand-held place, and when a single test strip is used for testing independently, the blank area 90 can be held by hand (referring to FIG. 3). There are 13 absorbent test pads, where each test pad includes a chemical reagent that is capable to test a substance in a liquid sample, and each test pad is arranged at intervals. For example, the white support sheet 114 as shown in FIG. 2 is the spacing area, and the distance between the test pads is generally 3-8 mm. The test pads are separated by some non-absorbent supports, without liquid flowing between them. Thus, when testing is needed, the liquid is dripped directly into the test pads sequentially and at intervals to complete the test. When there are multiple test pads, the blank area is left between every two test pads and is also the spacing area between the test pads.

When the conventional test strip is used, the liquid (for example, urine) is generally collected by a test tube with a depth equivalent to that of the test strip, the entire test strip is inserted into the test tube, each test pad is allowed to contact with the urine almost at the same time, taken out of the test tube and placed into a machine for reading (machine reading is performed based on the optical principle, for example, taking photos, analyzing the color blocks of the test pads, and automatically reading positive or negative results) or multiple test pads are compared with provided color cards with naked eyes to judge the color of each test pad and obtain the testing results. However, if such a test strip is directly operated by an inexperienced test subject and used especially for a case that no machine automatically reads the testing results when the test subject tests the urine at home, if an operation mode is also to insert the entire test strip, for example, one end of the test strip 100 (with leukocytes) is inserted into the test tube, taken out and then read, the operator needs to compare the color of 13 test pads with the color of the standard color card due to presence of 13 test indicators, and the color blocks of the standard card have many different meanings for each test indicator. For example, the specific values for positive conditions, the number of leukocytes, and the specific values of PH need to be read in a specified time. This is a challenging task for people who have no practical experience in home test, and they are almost impossible to complete the task. This is due to a fact that with many test indicators, corresponding color cards have various colors and different meanings, each test pad needs to be read within the specified or required time, and the overdue test (if any) causes the testing results to be invalid. For example, the first indicator (leukocytes) is usually read within 2 minutes, and the last indicator (glucose) needs to be read within 30 seconds. It basically takes 2-3 minutes to read from the first indicator to the third indicator, so the reading time from the first indicator to the third indicator exceeds the reading time of the last indicator early; generally, after the reaction of the test pad ends up, the last indicator is read too late to invalidate the testing results. Such an operation mode that multiple test indicators are exposed to liquid at one time and then the testing results are read sequentially is not suitable for home test and especially not suitable for people who have no operating experience or professional experience to operate and judge the testing results.

Therefore, the invention changes the conventional operation mode, and allows the test subject or operator to operate intermittently, that is, when one or two test pads are selected, after one test pad receiving the liquid completes test and reading, the liquid is dripped into the next test pad and its testing result is read, with an interval time or a waiting time during this test operation. Although the operation is inefficient, it is especially suitable for people who have no operating experience, such as home self-test. For example, the liquid is dripped onto one test pad, the dripped test pad reacts with the liquid, and then the testing results can be generally read within a specified time. For example, the dripped indicator for testing leukocytes can be read within two minutes, and the dripped indicator for testing nitrite is generally read within one minute, so the operator can smoothly complete the test and well record the testing results, has enough time to compare the testing results with each color block of the standard color card, and selects the color block having the closest color and record the testing results, without worrying about the reading time of the testing results of other test pads. After all, the liquid is not dripped onto other testing pads, and there will be no testing results for them. After the first test pad or the second test pad is tested, another two test pads, such as the test pad for testing urobilinogen and the test pad for testing microalbumin, are also tested intermittently. The urine sample is dripped into the two test pads, and after the test pads are tested, a third group of test pads including test pads for testing protein and PH value is tested. Thus, although the operation carried out according to the intermittent mode takes a longer time than the conventional mode, each testing result may be ensured to be effective and accurate. Therefore, when the intermittent mode mentioned here has a plurality of indicators, some test pads are selected for testing and the testing results thereof are read. After the selected some test pads are tested, other testing pads are tested again, unlike the conventional mode, all test pads are in contact with the liquid at one time, for example, one test pad may be selected for testing, and the next test pad is tested after the completion of the test.

"End of test" or "completion of test" mentioned here refers to a testing process, for example, this testing process may include color change (this time occurs quickly) displayed by the reaction of the test pad from the liquid in contact with the test pad, and may also comprise comparing the color of the test pad with that of the standard color card, and then the testing result is acquired and read according to the color comparison. The overall process above is the end of test, and certainly, the process may also include the time for recording the testing result after reading the testing result. That is to say, after the end of test or the completion of test of one test pad, the next test pad starts to be tested, then the test is end or completed. Actually, two test pads are not in contact with the sample simultaneously and have the interval waiting time, and thus the inexperienced home operator can perform the operation.

In another embodiment, for example the test strip as shown in FIG. 2, ten selected test pads are disposed on the non-absorbent support sheet 200, and the test indicators corresponding to each test pad are as follows: the test pad 101 for testing leukocytes, the test pad 102 for testing nitrite, the test pad 103 for testing urobilinogen, the test pad 105 for testing protein, the test pad 106 for testing PH values, the test pad 107 for testing blood, the test pad 108 for testing specific gravity, the test pad 111 for testing ketone, the test pad 112 for testing bilirubin and the test pad 113 for testing glucose. The testing mode can also be performed according to the method described above, thus self-testing at home can also be performed, and both the experienced operator and inexperienced operator can complete the test.

In some embodiments, to arrange the testing sequence reasonably, allow the effective testing time that the test indicator reads the testing result to be shortened in turn, or the time for reading the testing result to be effective is shortened gradually. In other words, the test pads are sorted in turn on the support sheet according to the time for reading the testing result effectively from the longest time to the shortest time, for example, the time for effectively acquiring the testing result of the first test indicator (the test pad for the first test) is the longest, and the time for acquiring the effective testing result of the last test indicator (the final or latest test pad) is the shortest. For example, the testing result of the test pad 101 for testing leukocytes has the reading time of 2 min, that is, the effective time from the liquid drop to the test reading is 2 min, or reading the testing result within 2 min is effective, while reading the testing result beyond this time is invalid, this is the longest time in all 10 or 13 test pads and ranks the first for the initial or first drop test, and after the first test pad is tested, the several subsequent test pads are intermittently tested. For example, the effective testing time of the following test indicators are as follows: 60 s for Nitrite, 60 s for urobilinogen, 60 s for protein, 60 s for PH value, 60 s for blood, 45 s for specific gravity, 40 s for ketone, 30 s for bilirubin and 30 s for glucose. Sequence shortening here is not the effective time that each test pad reads the testing result is strictly shortened, but overall shortening. The effective time of the testing result read by several test indicators is the same, but the general tendency is shortening.

It can be understood that the effective test time here is merely a specific embodiment and designed in advance, and the effective test time may be different due to reaction principles and different substrates and the like. But in any case, the preferred example is that the indicator with the longest effective test time is tested for the first time (initial), and the indicator with the shortest effective test time is tested finally, for example, the Glucose 115 is tested finally, with the effective test time of 35 s. Due to more test indicators, the effective test time of each test pad is different, and there is always the longest time or the shortest time.

"Effective test time" in the invention refers to the time from the sample dripping to test end and reading the testing result (including the record of the testing result), and the testing result acquired within this time scope is effective. If exceeding this time, the testing result is invalid, this is the chemical test, the color change of the test pad may be continuing, the test for reading the testing result in the specified time and completing the test is the effective testing result, and the test beyond this time is regarded as being invalid.

The biggest advantage for arranging in turn according to the effective test time is that a process for being familiar with the test must be experienced for the home self-testing, the longest effective time for reading the testing result is arranged foremost for the initial test, the shortest effective time is arranged in the end for the final test, thus there are several advantages as follows: due to the first operation, drop the liquid and wait for the color change of the test pad, then compare the test pad with the color block of the standard color card to acquire the testing result, actually this is a complicated process, specifically when comparing the color of the test pad with the color block on the standard color card, the color closest to the test pad needs to be selected to acquire the final testing result, which requires more time. Although the waiting time is the longest, the testing result or read testing result is also effective as long as the longest time is within the fixed effective time, thus reducing the operation difficulty and increasing the testing accuracy. Generally, there is always a timer on the side for reminding, the operator can basically understand the time for completing the first test indicator (test pad) from liquid dropping to the completion of test, or the time for the end of test. Thus, the completion time of the end of test of the next several test pads has a reference, for example, the time for the first or initial test indicator from the liquid dropping to the end of test is 60 s, then the testing for Nitrite with 60 s of effective testing result can be merely selected when selecting the next indicator for testing. However, if it has been found that the time of the first test indicator from the liquid dropping to the end of test is 25 s or 30 s, Nitrite and urobilinogen can be selected when performing the next test, after all, liquid can be separately dropped on the two test pads for testing the Nitrite and urobilinogen in turn according to the equal distribution, and then accurate reading can be carried out within 60s of effective time. After all, these test pads all react relatively independently, with the selectable operation sequence. In contrast, if the testing for the first test pad takes a long time, such as 110 s, a suitable time is selected for testing the next test pad under time consideration or plan before the next test pads, for example, 80-60 s of effective test time is selected, rather than the short effective test time, such as 30-35 s of test pad. Therefore, one or two indicators may be probably estimated next for testing according to the time spent to operate the first test indicator, after all, different operators spend different amounts of time, some careful operators may take a longer time, while some experienced operators with medical commonsense and good eyesight may take a shorter time. Thus, the testing process for the several indicators next may be arranged reasonably. After all, populations performing home testing are diversified, such as extroversion, caution, different visions and different educational levels. So, in some embodiments, the several indicators tested finally have the shortest effective test time, while the first test indicator has the longest effective test time. Thus, the operator's habit from not being familiar with the test to being familiar with the test can be ensured, in addition, this test has better adaptation and compliance from the longest effective time to the shortest effective time, which conforms to the operation habit and has more friendly use.

The above means that a plurality of test indicators are arranged on a non-absorbent support sheet. Of course, it can be understood that the non-absorbent support of the test strip can have 10 test indicators, 13 test indicators, and sometimes only one test indicator, for example, any one of 1-15 test indicators or 2, 3, 4, 5, 7, 10, 12 or 15 of any 15 test indicators, and the test pads corresponding to these test indicators are fixed onto the non-absorbent support sheet sequentially or at intervals, and can also be arranged from the longest time to the shortest time according to the effective test time.

If there are only test strips, when the liquid sample is dripped into the test pad, as shown in FIG. 3, for example, it is generally necessary to suck the liquid sample with a pipette, and then drip 1 drop or 1-2 drops of the liquid sample into the test pad. Although such a dripping requirement is made, many drops of the liquid sample are often dripped. In order to solve this problem, a piece of absorbent material 40 is placed under the test strip and absorbs the excess liquid sample so as not to flow to the adjacent test pad. For example, as shown in FIG. 2, when the liquid sample is dripped into the test pad 101 for testing leukocytes, it is not expected that the liquid sample will also contact with the test pad 102 for testing nitrite, and the excess liquid sample will flow onto the absorbent material 40. In addition, when the test pad only needs one drop of the liquid sample, sometimes a few more drops of the liquid sample are dripped due to the unfamiliarity of the operator, and these excess liquid samples will be absorbed by the absorbent material 40. Because each test pad has the saturated absorption capacity, if it absorbs the excess liquid sample, the testing results are affected; therefore, the excess liquid samples are expected to be absorbed without affecting the testing results. Some test pads have the saturated absorption capacity of just one drop, for example, 40-50 µL. As shown in FIG. 3A and FIG. 3B, it is a basic operating process. Firstly, the urine cup 10 is used to receive the urine sample; secondly, a test strip 200 is taken out from a packaging bag 20; thirdly, each test pad of the test strip is compared with a leftmost color strip 32 (a calibration strip, which is negative and has no color change) of a standard color card 30. In this case, if the color of each test pad is consistent with that of the color strip 32 of the standard color card, the test strip is valid; and if the color of any test point is different from the color of the color block on the color card, the test strip is invalid and the test cannot be performed. Therefore, the color of the leftmost color strip 32 on the color card is the same as the color of the test pad that has not started to react. If the color of the test pad on the test strip changes, the test strip is invalid; for example, the wet or contaminated test strip cannot be used for testing, so it should be abandoned or discarded. When it is judged that the test strip 200 is valid, the test strip 200 is placed on the surface of the absorbent paper 40, the liquid sample is absorbed from the urine cup with a pipette 50, a drop of the liquid sample is added to the first test pad 101, the color of the first test pad is compared with that of the color block of the corresponding test indicator of the color card 30, and then the testing results are read within the valid time. The first test pad 101 is tested, followed by the next test pad 102; and the test operation from the first test pad to the ninth test pad 112 is repeatedly performed in such a way; after the first test pad 101 is tested, the urine sample is dripped into the second test pad 102, and the testing results are judged within the effective time (for example, 60 seconds). When glucose is tested, a drop of the urine sample needs to be dripped, and the dropper is turned to spread the urine sample on the surface of the test pad, such that the urine sample can wet the test pad, and then the testing results are read. This is because the test pad of the test indicator contains a hydrophobic material and the urine sample is not easy to spread, it is necessary to spread the urine sample on the test pad and read the testing result within 35 seconds.

Although a single test strip (FIG. 1 - FIG. 2) can be used to complete the test according to the method shown in FIG. 3, there are still some disadvantages, for example, the distance between the test pads is relatively small, and sometimes, when the liquid sample is dripped, it may be dripped onto the test pad that is undesired to be tested, or when the excessive urine sample is dripped onto the test pad, it will flow to the adjacent test pads; although the gap between the test pads is a partially non-absorbent support sheet, the urine sample will still flow to the adjacent test pads, which will lead to mutual interference in the test and also affect the final testing effect. For example, when the urine sample is dripped to the first test pad 101, it may be also dripped into the second test pad 102. In this case, for the inexperienced home operator, such dripping will cause worry and misjudgment, and eventually the operator will not perform the test, such that the test is unfavorable and inconvenient. These unfavorable operations are mainly caused by many test pads and many test items, and none of them are caused by people with professional experience. In addition, at the end of each test, the color of the test pad needs to be compared with the color of a separate color card, and such operation is actually complicated. There are 10-13 test indicators distributed on the color card, and the depth of the color block of each test indicator is different, which is easy to cause confusion; such confusion often occurs when the color of the test pad is compared with that of the color card, and eventually wrong results are caused.

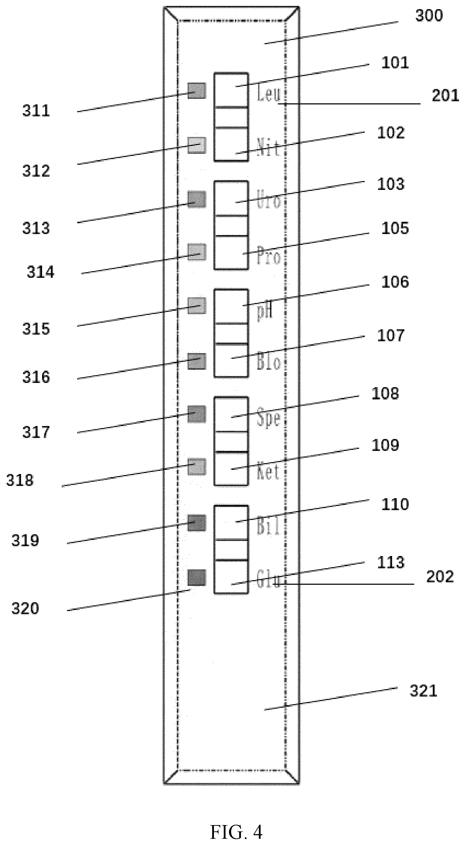

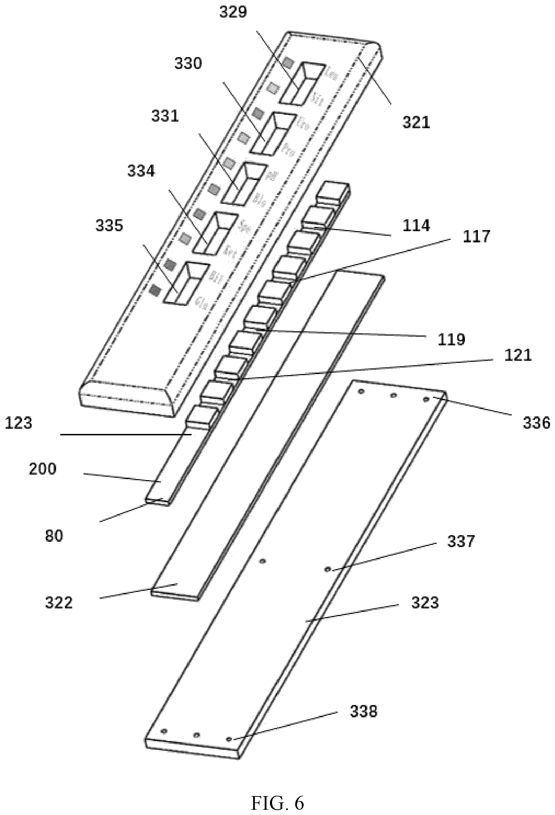

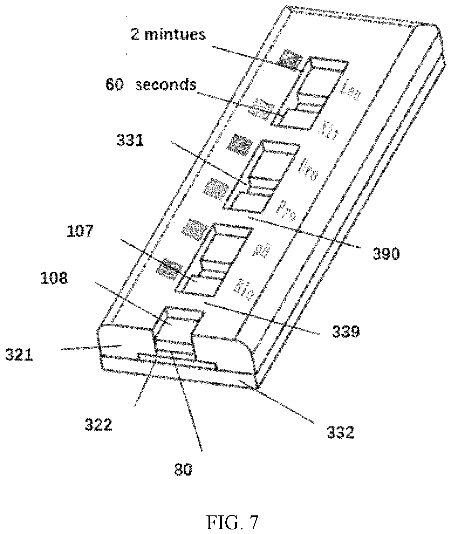

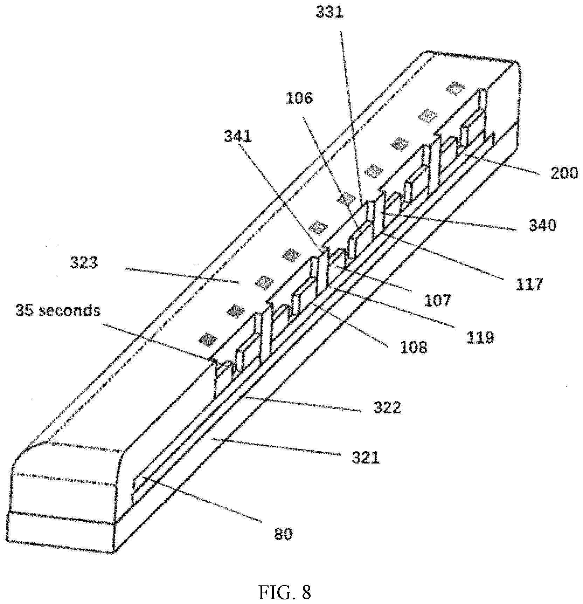

In order to obtain better test experience, especially for home test experience, the invention provides another design. In an embodiment as shown in FIG. 4 - FIG. 8, a lower board 323 and an upper board 321 are provided, the lower board is provided with insertion holes 338, 336, and the upper board is provided with insertion pins, such that the upper board and the lower board are combined to form a device. A plurality of windows, such as windows 324, 325, 326, 327, 328, are arranged on the upper board, two test pads are arranged in each window, and the first test strip is still a non-absorbent support sheet 200, and a plurality of absorbent test pads 101, 102, 1303, 105, 106, 107, 108, 109, 110, 113 are arranged on the non-absorbent support sheet. There is a gap 114, 115, 116, 117, 118, 119, 120, 121, 122 between the test pads on the test strip, with a total of 9 spacing areas (as shown in FIG. 2) having a same distance between them. In addition, an absorbent filter paper 322 is arranged under the support sheet 202. In some embodiments, partition structures 340, 341 are arranged on the upper board 321, and these partition structures extend downward from the areas 339, 390 between windows and contact the spacing areas 117, 118 on the non-absorbent support sheet, which has a squeezing effect on these spacing areas. Therefore, the test pad can be in a relatively independent space by virtue of the windows and the partition structures. For example, the test pads are grouped and arranged in the window at intervals, as shown in FIG. 8. However, the partition structures are clamped on the spacing areas on the test strip, for example, there are two test pads 106, 107 in the window 331. The partition structures 340, 341 are clamped on the spacing areas 117, 119 from ends of the test pads. Accordingly, the two test pads are located in the same window. In this way, every two test pads are divided into different windows; 10 test pads, if any, are respectively divided into five windows; and the windows are separated by the spacing structure, such that their test pads are more independent and separated from a spatial structure. In a specific way as shown in FIG. 4 - FIG.8, every two test pads are arranged in a window, and the windows are separated by the partition structure. The specific way is that barrier strips 340, 341 are arranged on the spacing areas 117, 119, and the width of the barrier strips is substantially equal to that of the spacing areas 117, 119. Specifically, the partition structures are arranged between the windows, and the ends of the partition structures cover the spacing areas 117, 119 between the test pads on the test strip, such that the first test pads 301, 302 are independently provided in each window, and five independent windows are provided and respectively have two independent test pads. In other words, because there are the partition structures between the windows, no liquid flows between the windows. Thus, the operator only needs to drip the liquid into the test pad in the window, and the window has a certain depth, so it is unlikely to drip the liquid into the adjacent window during liquid dripping. In addition, even if the excess liquid is present in the first window, it will not flow to the inner surface of the adjacent window because liquid flowing is blocked by the partition structure.

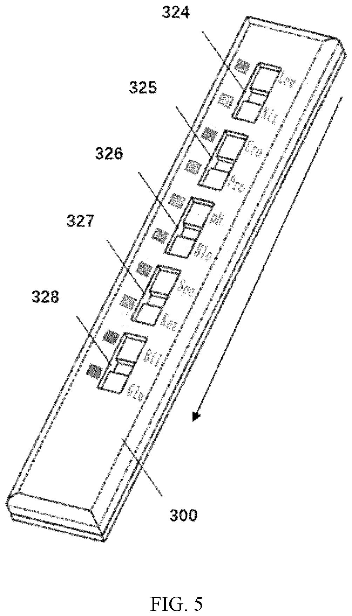

Of course, in some embodiments, a filter paper 322 is arranged under the support sheet and can absorb the excess liquid sample. In order to better make the excess liquid flow to the filter paper, the window is slightly wider than the test strip or the non-absorbent support sheet, and there is a gap 331 between the window and the test strip. When the excess liquid exists, it is absorbed through the gap and then flows to the filter paper. In some embodiments, some color blocks can be arranged on both sides of the window. For example, as shown in FIG. 4, negative result color blocks 311, 312, 313, 314, 315, 316, 317, 318, 319, 320 are arranged on the left side of the test pad corresponding to the window, and positive color blocks 201, 202 (not shown) are arranged on the right side (with test item names) thereof. When the liquid is dripped into the first test pad 301, the color of the first test pad is directly compared with the color of the left and right color blocks within the specified time to effectively read the testing results. The testing results are judged to be positive or negative testing results; and if the testing results are positive, expected further test is made for validation. If the color of the first test pad is close to the color of the left color block, this means that the color has not changed and the testing result is negative. If the color of the first test pad is the same as or deeper than the color of the left color block, the testing result is positive. Of course, the test pad in each window needs to be observed whether its color is kept consistent with the color of the left color block before test. If the color of the test pad is kept consistent with the color of the left color block, the test pad is valid. In the embodiment, the test indicators on the test strip are the same as those on the test strip shown in FIG. 2B. However, the color blocks are directly printed on the window, it is not necessary to compare the test strip with the color card with many different color blocks when the testing results are judged, thereby avoiding confusion. In some embodiments, the test pads are also arranged in the above order, their testing order is not changed substantially, and the sequential and interval test is still adopted. For example, as shown in FIG. 5, the test pads with the time to effectively read the testing results up to 2 minutes are firstly tested, and the test pads with the short time to effectively read the testing results are tested. The test of the embodiment has the following advantages: firstly, multiple test pads are divided into several independent units through the windows; the tests for each unit are independent, thus avoiding improper liquid dripping; and the liquid is separately dripped into each window, thus avoiding the wrong order of liquid dripping, for example, the liquid is originally dripped to the first test pad, but mistakenly dripped to the second test pad. Secondly, if the excess liquid is dripped onto the first test pad 101 or the second test pad 102, it will not flow onto the third test pad 103, which ensures the test pad 103 is independently tested and will not be disturbed by the excess liquid from the second test pad 102 (due to the function of the partition structure). It can be understood that in presence of an individual test strip, when an operator drips the liquid onto the second test pad 102, sometimes the liquid may be dripped onto a partition 114 between the second test pad 102 and the third test pad 103. Although the liquid dripped onto the partition 114 is not absorbed by the support sheet, it is easy to contact with the dry test pad, and the two test pads are kept in liquid communication through the liquid located on the partition 114, thus causing cross-contamination between adjacent test pads. Then, a partition or blocking structure is arranged on the partition 114, flowing of the liquid between the second and third test pads is cut off. Thirdly, negative color blocks 311-320 are tested near the window and the negative color blocks 311 are directly printed near the test pad. Before and after the test, the testing results can be judged by directly referring to the color blocks, instead of comparing the test strip with the standard color block. As the testing device is provided and the assembling volume of the upper card and the lower card is larger than that of a single test strip, the test strip is more difficult to judge the testing results when compared with the color card.

The testing device as shown in FIG. 4 - FIG. 8 may not be packaged in an aluminum foil bag, or a plurality of testing devices may be put together, for example, in a large bag. In addition, when a plurality of test devices are put together, a test strip (as shown in FIG. 1 or FIG. 2) and a color card are placed in a kit. In order to verify the validity of the testing devices, a single test strip is directly compared with the verification color card of the color card. If the test strip is valid, this means that the test devices are also valid, and the test strip is especially suitable for use by many people in the family or people who often use it.

In addition, the test strip as shown in FIG. 3B is provided to facilitate the recording of testing results. When the color of the test pad is compared with the color of the color block of the color card, the color block closest to the color of the test pad is selected and a circle is drawn on the color card to indicate the testing results.

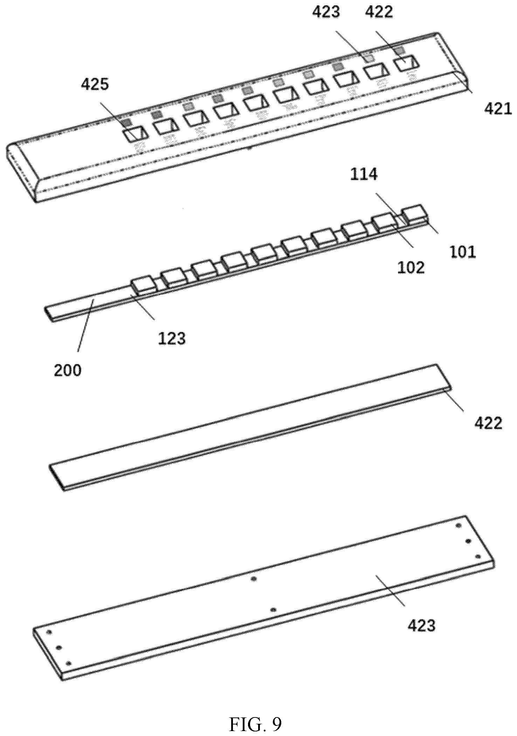

In other embodiments, for example, as shown in FIG. 9, a single test pad 101 of the test strip is located in one window, and if there are ten test indicators, ten separate windows are provided for ten test pads, and the ten test pads are located in the ten windows respectively, such that one test pad is provided in each window. In addition, nine partition structures are provided (the specific way is the same as that shown in FIG. 4 - FIG. 8), and each partition is located in the spacing area between the test pads, such that each test pad can exist independently, and the dripping liquids will not flow with each other to affect the testing results. During operation, the liquid is directly dripped into the window, and the result is directly read within the effective time. This means that each test pad is tested independently, and the liquid is dripped through the window, thereby ensuring that the liquid will not be dripped into another window. Similarly, the color block, such as a negative color block or a positive color block, can be printed near each window, such that the color of the test pad is conveniently compared with that of the color block on the window to determine the testing results, for example, the testing results are negative results or positive results. For example, the positive color block is printed on the left side of the window, and the negative color block is printed on the right side of the window. When the color of the test pad is close to that of the negative color card, the negative color block is directly marked, indicating that the testing result of the corresponding test pad is negative, and on the contrary, the positive color block is marked, indicating that the testing result of the test pad is positive.

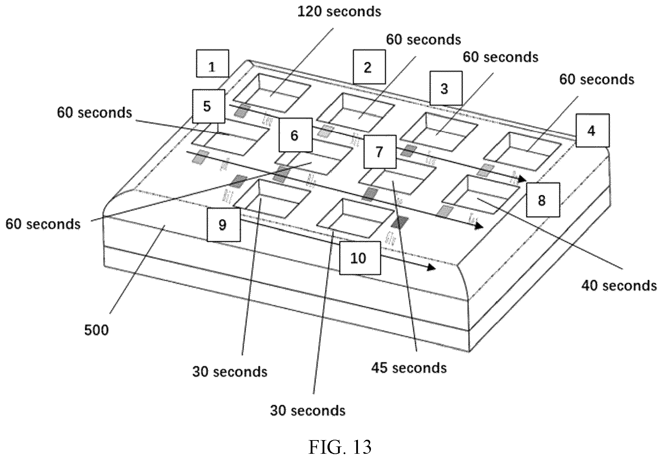

As shown in FIG. 4 - FIG. 8 and FIG. 9, the test pads are arranged in such a way that the first test pad has the longest effective testing time and the last test pad has the shortest effective testing time. In some embodiments, in order to make home test more convenient, the testing sequence can be marked next to the window, as shown in FIG. 13. For example, numbers 1-10 can be printed near the window of the testing device, where the number 1 indicates first test. Of course, the time to effectively read the testing results, for example, 2 minutes, 60 seconds and 35 seconds, the time to effectively read the testing results after the liquid is dripped into the test pad, for example, 1-2 minutes, 2-60 seconds, 3-60 seconds and 10-35 seconds, and words for reminding the operator of the test pads and the time to effectively read the testing results can also be printed near the window of the testing device, such that the information given is clear and too many operating instructions are basically not required. Moreover, each test pad can be operated independently, and even if other things lead to the delayed test in the midway, the test pad can still continue to be tested. After all, this means that each test pad can be tested independently and the testing results can be judged independently. Thus, the test pad is very convenient to test at home and brings greater convenience.

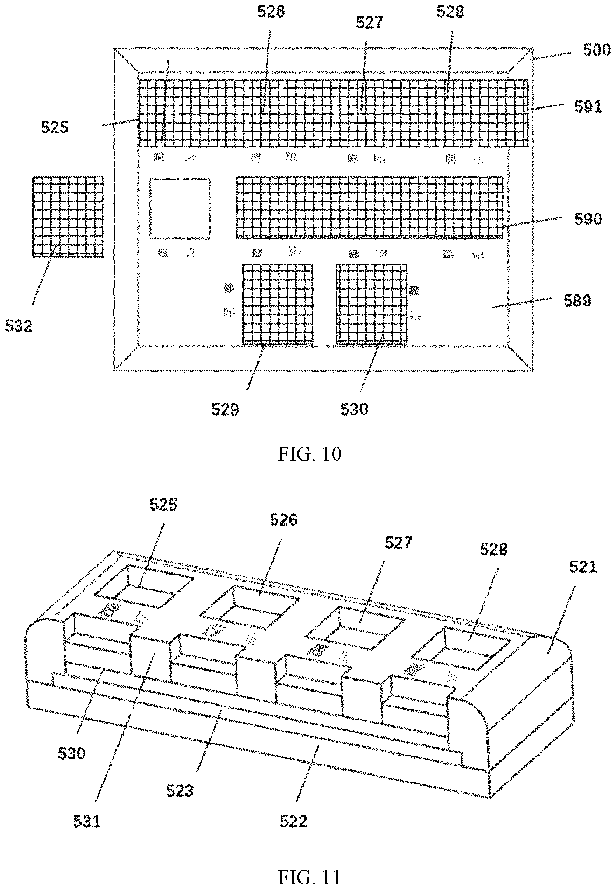

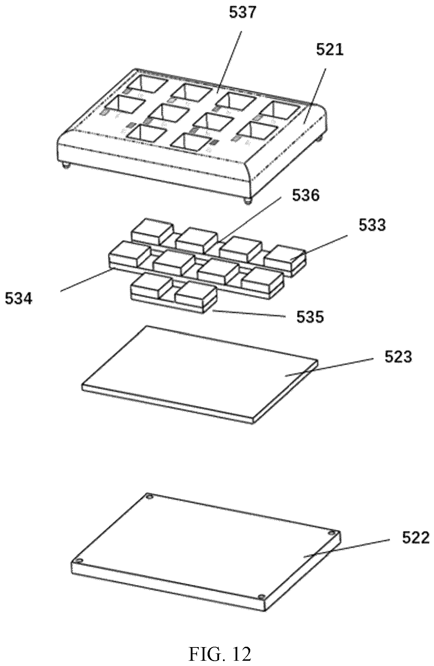

In other embodiments, three test strips are provided and respectively have two or more test items. As shown in FIG. 12, three test strips 533, 534, 535 are provided, the first test strip has two test pads, the second test strip has four test pads, the third test strip has four test pads, each test pad is corresponding to a test item or a test indicator, and the corresponding indicator of each test pad is different. Correspondingly, a lower board 522 and an upper board 521 are provided. The upper board is provided with a plurality of windows, where one test pad is accommodated in each window and one partition structure is arranged between the windows, and the partition structure is arranged between the spacing areas of the test pad, such that one test pad is accommodated in the window. For example, as shown in FIG. 11, four test pads are arranged on the test strip 525, and there are spacing areas between the test pads. Three spacing areas are provided, and three partition structures are arranged on the window to separate the four test pads and make them independently located in the window. The test strip is also a non-absorbent support sheet 530 with four absorbent test pads. Of course, in a preferred embodiment, a plurality of color blocks may be arranged near the window, including positive color blocks and negative color blocks; the positive color blocks with different color depths can be arranged, thereby learning about whether the specific test indicators on the test pad are positive and the specific approximate content thereof. Of course, the name of the test indicator of each test pad can be identified near each window of the device as shown in FIG. 4 - FIG. 12, for example, the abbreviation of Leu for the number of leukocytes, such that the operator can learn about the analyte tested by the test pad or the name of the test indicator.

In some embodiments, a non-absorbent film covers the window or multiple windows 525, 526, 527, 528 of the testing device as shown in FIG. 10. The non-absorbent transparent film covers the window, which not only prevents the test pad in the window from moisture but also prevents the liquid from improper dripping. During testing, the non-absorbent film can be uncovered from one end to expose a window test pad, and then the liquid is dripped into the window where the test pad is exposed for testing. Because the adjacent window is covered with the transparent film, the liquid will not be dripped into the adjacent window by mistake. FIG. 10 is taken as an example, the surfaces of three rows of windows are covered with the transparent films. When four test indicators in a third row 591 need to be tested, the transparent films are uncovered from the surface of the window in the third row to expose the first test pad (leftmost) of the third test strip 526, the urine is dripped into the first window, and then the testing results are read within 2 minutes. In this case, other windows in the third row are covered with the films, and the windows in a second row 590 and a first row 589 are also covered with the films. Therefore, during operation, the liquid can only be dripped onto the test pad for testing Leu of the first window. Preferably, these films can be black opaque films to prevent the test pad from oxidation by light, the black films are uncovered to expose the window and the test pad, and the other windows are still covered with the black films, such that the window where the liquid is dripped can be more clearly indicated, the liquid will not be dripped into other windows, and after all, the windows are still covered with the films. When two test pads (for example, test pads for testing nitrite) are tested, the films on the window are uncovered to expose the test pad in the window such that the test pad can be tested. These films can cover all windows as a whole, or a single film can cover one window, for example, the film 532 covers the window for testing PH. Alternatively, the films 529, 530 respectively cover the windows where the two test pads on the test strip 535 are located. During testing, the film is individually uncovered for testing.

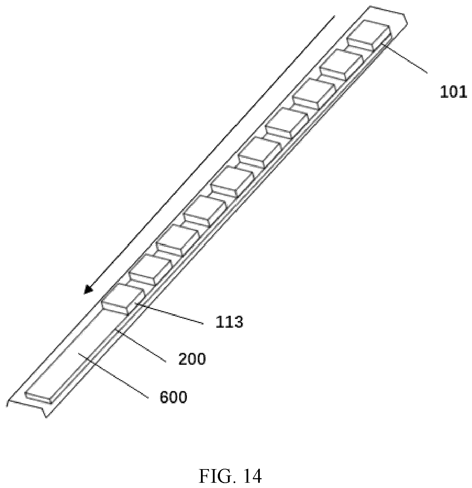

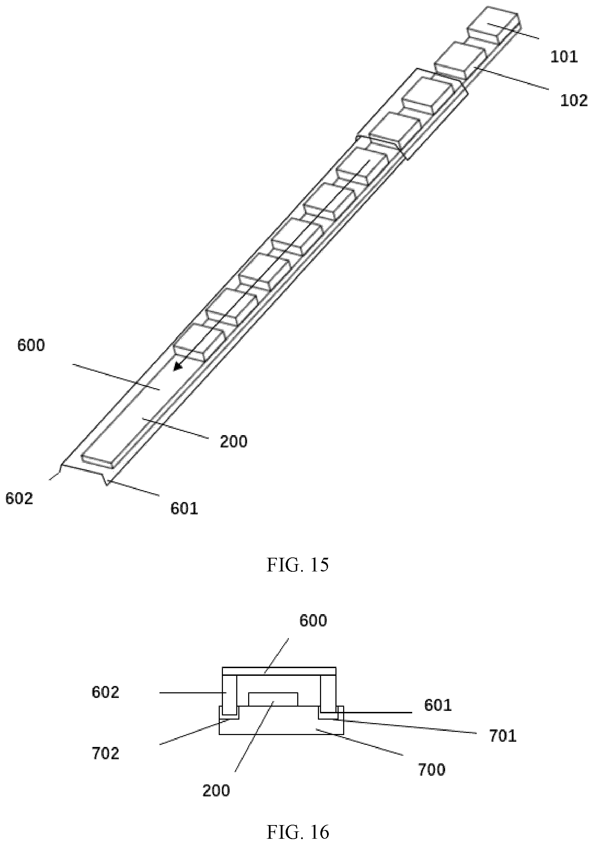

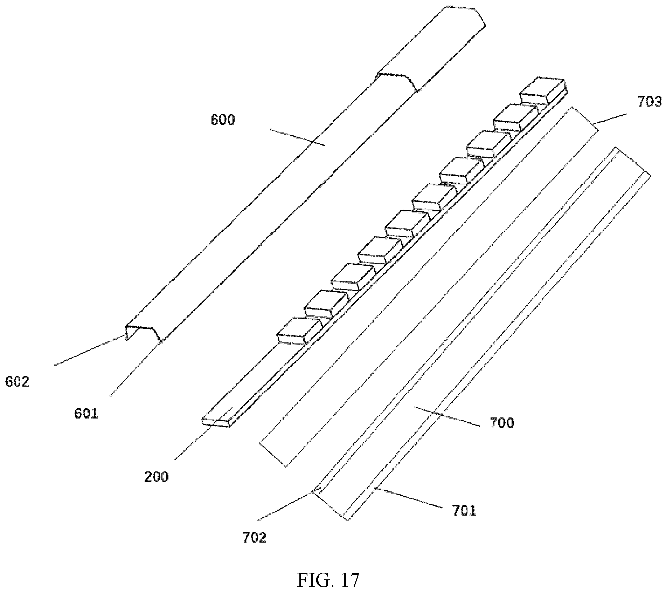

In other embodiments, as shown in FIG. 15 - FIG. 17, a test strip 200 is provided, a slidable cover 600 is arranged on the test strip, and the slidable cover covers the test pad. When testing is required, the test pad is gradually exposed for testing by sliding the cover 600 sequentially and at intervals. For example, as shown in FIG. 14, the test pad 101 is exposed by sliding the cover to test the test indicator of the test pad 101. After the test, the test pad 102 is exposed by again sliding the cover to test the test pad 102. For more convenient operation, the bottom board 700 is provided on the test strip and has chutes 701, 702, while the cover has two sliding rails 601, 602 and has two edges as the sliding rails 601, 602. The chutes are arranged in the bottom board, the sliding rails can slide in the chutes, the absorbent material is arranged on the bottom board and located below the support sheet of the test strip 200, and the cover 600 slides longitudinally. The bottom board, the absorbent paper and the test strip will not move, such that the test pads are exposed one after another for testing, and cross contamination caused by dripping the liquid onto the adjacent test pads is avoided.

All of the embodiments are belong to the pricinple of the invention.
1. A testing device, comprising:
   a test strip, wherein the test strip comprises a non-absorbent support sheet on which at least two or more absorbent test pads are arranged, each of the absorbent test pads is separated by the non-absorbent support sheet, the absorbent test pads comprise a reaction reagent capable to test an analyte in a urine sample or test the property of the urine sample; and
   an absorbent material, wherein the absorbent material is arranged under the non-absorbent support sheet; and when the urine sample is dripped into one of the at least two or more absorbent test pads, the absorbent material is capable to absorb the redundant urine sample, whereby preventing the redundant urine sample from flowing to other test pads into which the urine sample is not dripped.
2. The testing device according to clause 1, wherein the absorbent test pads are dry before the urine sample is dripped, and get wet after the urine sample is dripped, such that the absorbent test pads are capable to give rise to a color change to exhibit whether the analyte in the urine sample is negative or positive.
3. The testing device according to clause 2, wherein the urine sample is dripped into the test pads sequentially and at intervals.
4. The testing device according to clause 3, wherein the sequential and interval application of the urine sample refers to the first application of the urine sample from a longest time to effectively read testing results.
5. The testing device according to clause 4, wherein the test strip is further provided with a cover board that covers a testing area and is capable to longitudinally move along the test strip, and when the urine sample is dripped into the test pads sequentially and at intervals, the test pads are exposed out of the cover board sequentially and at intervals.
6. The testing device according to clause 4, further comprising flexible films covering the test pads, wherein when the urine sample is dripped into the test pads sequentially and at intervals, the flexible films are uncovered sequentially and at intervals to expose the test pads into which the urine sample is to be dripped.
7. The testing device according to clause 4, further comprising an upper card with a window and a lower card as a bottom board, wherein the test strip is located between the upper card and the lower card, the test pads are located in the window, and the absorbent material is located on the lower card and under the non-absorbent support sheet.
8. The testing device according to clause 7, wherein one or two test pads are exposed out of each window, and the urine sample is dripped into the one or two test pads sequentially and at intervals through the window.
9. The testing device according to clause 8, wherein the upper card further comprises a spacing structure, and the spacing structure is located on a non-absorbent spacing area between the test pads, such that the one or two test pads are arranged independently in the window.
10. The testing device according to clause 9, wherein one test pad is exposed out of each window, the spacing structure extends out of each window and is located on the non-absorbent spacing area between each two test pads, such that one test pad is arranged independently in the window; the urine sample is dripped into each test pad through the window, whereby observing the color change of each test pad to judge whether the testing results are negative or positive.
11. The testing device according to clause 9, wherein a width of the window is larger than a width of the non-absorbent support sheet of the test strip, such that a gap is arranged between the window and the non-absorbent support sheet, and thus the redundant urine sample is capable to flow to the underlying absorbent material from the gap and be absorbed.
12. The testing device according to clause 7, further comprising a color card printed with color blocks with different color depths, wherein the color of the test pad is compared with that of the color card to obtain the number of the analyte in the urine sample or the property of the urine sample.
13. The testing device according to clause 12, wherein the color block is arranged on an upper board and close to the window.
14. The testing device according to clause 10, wherein ten test pads are provided in the testing area, each test pad comprises a chemical reagent that is capable to test the analyte in the urine sample or the property of the urine sample, and the ten test pads are respectively used for testing leukocytes, nitrite, urobilinogen, protein, PH value, blood, specific gravity, ketone and bilirubin in an order from a long time to a short time to effectively read the testing results.
15. The testing device according to clause 10, wherein thirteen test pads are provided in the testing area, each test pad comprises a chemical reagent that is capable to test the analyte in the urine sample or the property of the urine sample, and the thirteen test pads are respectively used for testing leukocytes, nitrite, urobilinogen, protein, PH value, blood, specific gravity, ascorbin acid, creatinine, ketone, bilirubin, and glucose in an order from a long time to a short time to effectively read the testing results.
16. The testing device according to clause 1, wherein the test pad comprises a chemical substance for testing urine glucose and a hydrophobic polymer.
17. A testing device, comprising: a test strip, wherein the test strip comprises a non-absorbent support sheet on which at least two or more absorbent test pads are arranged, each of the absorbent test pads is separated by the non-absorbent support sheet, the absorbent test pads comprise a reaction reagent, and the reaction reagent is capable to test an analyte in a urine sample or test the property of the urine sample; wherein the test pads are arranged in an order that the test pad with a longest time to effectively read testing results is firstly arranged, firstly dripped with the urine sample, and followed by the test pad with a shorter time to effectively read testing results than the longest time to effectively read testing results; wherein the test pad with a shorter time to effectively read testing results is used for secondly receiving the urine sample, and the urine sample is secondly received after the testing results of the first test pad are read.
18. A method for testing a urine sample, comprising: providing a device, wherein the device comprises a test strip, wherein the test strip comprises a non-absorbent support sheet on which at least two or more absorbent test pads are arranged, each of the absorbent test pads is separated by the non-absorbent support sheet, the absorbent test pads comprise a reaction reagent, and the reaction reagent is capable to test an analyte in a urine sample or test the property of the urine sample;
   intermittently dripping the urine sample to a plurality of test pads; after dripping the urine sample to each testing area, observing the color of the testing area, and comparing the color of the testing area with a color of a color card, to judge the content of the analyte in the testing area and complete the test.
19. The method according to clause 18, wherein after the test pad with a longest time to effectively read testing results is firstly tested, the test pad with a shorter time to effectively read testing results is tested.
20. The method according to clause 18, wherein the test pads on the test strip are arranged in an order from a long time to a short time to effectively read the testing results, such that the test pads are tested sequentially and at intervals.
21. The method according to clause 19, comprising: dripping the urine sample to a first test pad with a longest time to effectively read testing results; reading the testing results within an effective time; and then dripping the urine sample to a second test pad with a shorter time to effectively read testing results than the longest time of the first test pad.
22. The method according to clause 21, wherein ten test pads are provided on the test strip and arranged according to an order of receiving the urine sample as follows: a first pad for testing leukocytes, a second test pad for testing nitrite, a third test pad for testing urobilinogen, a fourth test pad for testing protein, a fifth test pad for testing a PH value, a sixth test pad for testing blood, a seventh test pad for testing specific gravity, an eighth test pad for testing ketone, a ninth test pad for testing bilirubin, and a tenth test pad for testing glucose, wherein after the urine sample is dripped into the first test pad, the testing result of the first test pad is read within the effective time of the first test pad; subsequently, the urine sample is dripped into the second test pad and the testing result of the second test pad is read within the effective time of the second test pad; and the testing results are read by repeating the order until the tenth test pad is tested.
23. The method according to clause 22, wherein the time to effectively read the testing results is 2 minutes, 60 seconds, 60 seconds, 60 seconds, 60 seconds, 60 seconds, 40 seconds, 35 seconds and 35 seconds according to an arrangement order of the ten test pads.
24. The method according to clause 22, wherein when the urine sample is dripped into the test pad for testing glucose, a dropper contacts with the test pad and gently rotates to spread the urine sample on the test pad.
25. The method according to clause 18, wherein the test strip is further provided with a cover board that covers a testing area and is capable to longitudinally move along the test strip, and when the urine sample is dripped into the test pads sequentially and at intervals, the test pads are exposed out of the cover board sequentially and at intervals.
26. The method according to clause 18, wherein the testing device further comprises flexible films covering the test pads, wherein when the urine sample is dripped into the test pads sequentially and at intervals, the flexible films are uncovered sequentially and at intervals to expose the test pads into which the urine sample is to be dripped.
27. The method according to clause 18, wherein the testing device further comprises an upper card with a window and a lower card as a bottom board, wherein the test strip is located between the upper card and the lower card, the test pads are located in the window, and the absorbent material is located on the lower card and under the non-absorbent support sheet.
28. The method according to clause 27, wherein one or two test pads are exposed out of each window, and the urine sample is dripped into the one or two test pads sequentially and at intervals through the window.
29. The method according to clause 28, wherein the upper card further comprises a spacing structure, and the spacing structure is located on a non-absorbent spacing area between the test pads, such that the one or two test pads are arranged independently in the window.
30. The method according to clause 29, wherein one test pad is exposed out of each window, the spacing structure extends out of each window and is located on the non-absorbent spacing area between each two test pads, such that one test pad is arranged independently in the window; the urine sample is dripped into each test pad through the window, whereby observing the color change of each test pad to judge whether the testing result is negative or positive.
31. The method according to clause 30, wherein a width of the window is larger than a width of the non-absorbent support sheet, such that a gap is arranged between the window and the non-absorbent support sheet, and the redundant urine sample is capable to flow to the underlying absorbent material from the gap and be absorbed.
32. The method according to clause 31, wherein the testing device further comprises a color card printed with color blocks with different color depths, wherein the color of the test pad is compared with that of the color card to obtain the number of the analyte in the urine sample or the property of the urine sample.
33. The method according to clause 32, wherein the color block is arranged on an upper board and close to the window.
34. The method according to clause 33, wherein the device is provided with a testing sequence label on each test pad to indicate that a testing person performs test sequentially and at intervals according to the label.
35. The method according to clause 34, wherein the device is provided with a time label to effectively read the testing results on each test pad to indicate that the testing person obtains the testing results according to the time label to effectively read the testing results.

All the patents and publications mentioned in the description of the invention indicate that these are public technologies in the art and can be used by the invention. All the patents and publications cited herein are listed in the references, just as each publication is specifically referenced separately. The invention described herein can be realized in the absence of any one element or multiple elements, one restriction or multiple restrictions, where such restriction is not specifically described here. For example, the terms "comprising", "essentially consisting of and "consisting of" in each example herein may be replaced by the rest 2 terms. The term "a/an" herein merely means "one", but does not exclude including 2 or more instead of including only one. The terms and expressions which have been employed herein are descriptive rather than restrictive, and there is no intention to suggest that these terms and expressions in this description exclude any equivalents, but it is to be understood that any appropriate changes or modifications can be made within the scope of the invention and appended claims. It can be understood that the embodiments described in the invention are some preferred embodiments and features. Any person of ordinary skill in the art can make some modifications and changes according to the spirit of the description of the invention. These modifications and changes are also considered to fall within the scope of the invention and the scope limited by independent claims and dependent claims.

## Claims

1. A method for testing a urine sample, comprising: providing a device, wherein the device comprises a test strip, wherein the test strip comprises a non-absorbent support sheet on which at least two or more absorbent test pads are arranged, each of the absorbent test pads is separated by the non-absorbent support sheet, the absorbent test pads comprise a reaction reagent, and the reaction reagent is capable to test an analyte in a urine sample or test the property of the urine sample;
intermittently dripping the urine sample to a plurality of test pads; after dripping the urine sample to each testing area, observing the color of the testing area, and comparing the color of the testing area with a color of a color card, to judge the content of the analyte in the testing area and complete the test.

2. The method according to claim 1, wherein after the test pad with a longest time to effectively read testing results is firstly tested, the test pad with a shorter time to effectively read testing results is tested.

3. The method according to claim 2, wherein the test pads on the test strip are arranged in an order from a long time to a short time to effectively read the testing results, such that the test pads are tested sequentially and at intervals.

4. The method according to any one of claims 1-3, wherein the device further comprises: dripping the urine sample to a first test pad with a longest time to effectively read testing results; reading the testing results within an effective time; and then dripping the urine sample to a second test pad with a shorter time to effectively read testing results than the longest time of the first test pad.

5. The method according to any one of claims 1-4, wherein ten test pads are provided on the test strip and arranged according to an order of receiving the urine sample as follows: a first pad for testing leukocytes, a second test pad for testing nitrite, a third test pad for testing urobilinogen, a fourth test pad for testing protein, a fifth test pad for testing a PH value, a sixth test pad for testing blood, a seventh test pad for testing specific gravity, an eighth test pad for testing ketone, a ninth test pad for testing bilirubin, and a tenth test pad for testing glucose, wherein after the urine sample is dripped into the first test pad, the testing result of the first test pad is read within the effective time of the first test pad; subsequently, the urine sample is dripped into the second test pad and the testing result of the second test pad is read within the effective time of the second test pad; and the testing results are read by repeating the order until the tenth test pad is tested.

6. The method according to claim 5, wherein the time to effectively read the testing results is 2 minutes, 60 seconds, 60 seconds, 60 seconds, 60 seconds, 60 seconds, 40 seconds, 35 seconds and 35 seconds according to an arrangement order of the ten test pads.

7. The method according to claim 6, wherein when the urine sample is dripped into the test pad for testing glucose, a dropper contacts with the test pad and gently rotates to spread the urine sample on the test pad.

8. The method according to any one of claims 1-7, wherein the test strip is further provided with a cover board that covers a testing area and is capable to longitudinally move along the test strip, and when the urine sample is dripped into the test pads sequentially and at intervals, the test pads are exposed out of the cover board sequentially and at intervals.

9. The method according to any one of claims 1-7, wherein the testing device further comprises flexible films covering the test pads, wherein when the urine sample is dripped into the test pads sequentially and at intervals, the flexible films are uncovered sequentially and at intervals to expose the test pads into which the urine sample is to be dripped.

10. The method according to any one of claims 1-7, wherein the testing device further comprises an upper card with a window and a lower card as a bottom board, wherein the test strip is located between the upper card and the lower card, the test pads are located in the window, and the absorbent material is located on the lower card and under the non-absorbent support sheet.

11. 28. The method according to claim 10, wherein one or two test pads are exposed out of each window, and the urine sample is dripped into the one or two test pads sequentially and at intervals through the window.

12. The method according to any one of claims 10-11, wherein the upper card further comprises a spacing structure, and the spacing structure is located on a non-absorbent spacing area between the test pads, such that the one or two test pads are arranged independently in the window.

13. The method according to any one of claims 10-12, wherein one test pad is exposed out of each window, the spacing structure extends out of each window and is located on the non-absorbent spacing area between each two test pads, such that one test pad is arranged independently in the window; the urine sample is dripped into each test pad through the window, whereby observing the color change of each test pad to judge whether the testing result is negative or positive.

14. The method according to any one of calims 10-13, wherein a width of the window is larger than a width of the non-absorbent support sheet, such that a gap is arranged between the window and the non-absorbent support sheet, and the redundant urine sample is capable to flow to the underlying absorbent material from the gap and be absorbed.

15. The method according to any one of claims 1-14, wherein the device is provided with a testing sequence label on each test pad to indicate that a testing person performs test sequentially and at intervals according to the label.

16. The method according to any one of claims 1-15, wherein the device is provided with a time label to effectively read the testing results on each test pad to indicate that the testing person obtains the testing results according to the time label to effectively read the testing results.
